# EUROPEAN PATENT SPECIFICATION

(11) **EP 0 952 825 B1**
(45) Date of publication and mention of the grant of the patent: **14.08.2002**
(21) Application number: 96941740.1
(22) Date of filing: 05.12.1996
(51) Int. Cl.: A61K 9/70, A61K 9/00, A61N 2/08, A61F 13/02, A61H 39/00

(54) **PATCH CARRYING A PARTICULATE MATERIAL AND A MAGNET**
PFLASTER ENTHALTEND TEILCHENFÖRMIGES MATERIAL UND EINEN MAGNETEN
TIMBRE PORTANT UN MATERIAU PARTICULAIRE ET UN AIMANT

(30) Priority: 05.12.1995 GB 9524865; 07.05.1996 GB 9609478
(43) Date of publication of application: 03.11.1999
(73) Proprietor: Jehan, David, Birmingham B15 1PT (GB)
(72) Inventor: Jehan, David, Birmingham B15 1PT (GB)
(74) Representative: Carpenter, David
(86) International application number: GB9603009
(87) International publication number: WO9720549

(56) References cited:
- EP-A- 0 081 109
- WO-A-84/00298
- WO-A-95/09591
- GB-A- 2 052 993
- DATABASE WPI Section Ch, Week 8404 Derwent Publications Ltd., London, GB; Class B07, AN 84-019994 XP002027228 & JP 49 020 321 A (TANAKA E) , 22 February 1974
- DATABASE WPI Section Ch, Week 8447 Derwent Publications Ltd., London, GB; Class B07, AN 84-290855 XP002027229 & JP 59 055 824 A (UNO K) , 31 March 1984
- DATABASE WPI Section Ch, Week 8433 Derwent Publications Ltd., London, GB; Class A96, AN 84-203893 XP002027230 & JP 59 116 214 A (MAGNE KK) , 5 July 1984

## Description

This invention relates to patch structures for transdermal therapy.

Patches are well known for providing treatment of varying effects directly through or on the skin. The most well known being the nicotine patch and hormone replacement patches. Other examples include patches containing metallic sheet material, such as copper (as disclosed in *WO95*/*09591*) or magnetic therapy (as disclosed in GB Patent *1596314*). Existing patches have a number of disadvantages. Nicotine patches and the like tend to release their active ingredients relatively quickly and thus it is not easy to maintain either a controlled dose, or a slow release. Also in some patches the active ingredients are in powder form and can be partially or totally covered by the adhesive used to adhere them to the patch, thereby restricting their effect.

In other cases, such as simple dressings the active ingredients may be applied in liquid form and these tend to dry in an uncontrolled manner. Patches incorporating metallic strips or sheets have an added disadvantage in that they do not always provide a high or uniform contact with the skin, in that the sheets do not always follow the contour of the body and thus only partial contact, and sometimes very low contact is achieved. Also the metal may have sharp edges which can cause injury. In many cases sweat is relied upon to provide a carrier to the skin can actually accumulate and form a barrier as can the presence of hair. It has also been found that the adhesives used to bond the active ingredients to the patch can irritate more sensitive skin types, rendering the use of patches impossible in some cases.

It is one object of the present invention to overcome the disadvantages of the above as well as to provide a more versatile and universal patch structure.

According to the present invention there is provided a patch for transdermal therapy comprising at least one layer of sheet material having on at least one surface thereof at least one magnet and, particulate copper or an alloy thereof on said one surface of said layer in conjunction with said magnet.

Preferably there is provided particulate zinc or an alloy thereof in conjunction with said particulate copper and said magnet.

The magnet may be a rare earth magnet and may be coated, with plastics material or with zinc, copper or suitable alloy thereof.

Preferably the particulate material is of a shape which does not abrade the skin, for example it may be provided with a rounded surface and may be of any suitable shape such as spherical.

The sheet material provides a carrier for the particulate material but may also be an active ingredient itself. The carrier may be a pad of a foam or other elastomeric material. The sheet material may be made of any convenient material used in or suitable for skin contact, for example hypo-allergic material for prolonged use, and all or part of one surface of the sheet material may carry an adhesive for applying to the skin. The sheet material may be of more than one layer in that there is provided a first layer on which the particulate material is carried or embedded, and a second or subsequent layer carrying the first layer and adapted to overlie the skin. The sheet material may be any convenient shape, such as circular or oval or the like.

The particulate material may be of any suitable material including or incorporating one or any combination of the following materials; metallic, ceramic, gelatinous, or of soluble plastics, natural sponge, cellulose, or any suitable material capable of carrying a required substance for therapy. The particulate material may be formed from any combination of materials. Thus a combination of metallic, magnetic and ceramic particles, for example may be used to enhance performance or to create a variety of effects.

The particulate material may be in any convenient shape, such as spherical, or size, such as between 45 to 1000 micron. For example the particulate material may be of any shape both regular and irregular, symmetrical or non-symmetrical. Some shapes, such as cylinders may be bound or co-joined to form tufts. The particulate material may be solid, hollow or porous, and may itself be the active ingredient or act as a carrier for said ingredient. The particulate material may be arranged in any formation as suits the particular application. It will be seen that where certain shapes of material are used spaces will naturally form between. Alternatively the material can be arranged so that definite spaces are provided. It is an advantage that the spaces tend to fill with sweat enhancing contact with the skin.

The particulate material may be bonded to the sheet material using any standard adhesive. It will be seen that the particulate material may extend from the adhesive to ensure said adhesive has no or minimum contact with the skin. The adhesive may be applied as a layer or may be discrete. The bonding material may be absorbent or waterproof, and may itself carry an active ingredient.

Generally a patch having this structure will have on a single surface the area carrying the particulate material thereon together with an area bearing the adhesive for adhering to the skin. Alternatively, where the user has sensitive skin the patch carries no additional adhesive and is held on the skin by other means. One solution is to provide adhesive on the surface of the patch remote from the active ingredients so that the patch can be adhered inside clothing, or to the inside of a band such as a wristband or a waistband or the like. The need for adhesives can be reduced or eliminated by trapping the particulate material between a permeable layer of sheet material and the carrier material to ensure that the re can be no direct contact between the skin and the adhesive bonding the particulate material. It is a particular advantage of elongate particles arranged in tufts or in tuftlike array the skin can be kept free from the adhesive.

It is to be noted that the invention is not restricted to relatively small patches that are normally envisaged, but may be employed on a larger scale depending on the application or surface area.

The particulate material itself may be self-bonded where the active material is flexible or elastic such as an impregnated cellulose.

In one embodiment of the present invention a patch structure comprises a layer of sheet material carrying, on at least one area of one surface thereof, a plurality of spherical particles of zinc and of copper arranged in a formation so that the particles are in close proximity but may or not be touching. When placed on the skin, either by conventional adhesive or by other means described elsewhere very small electromagnetic impulses are generated, useful in the treatment of skeletal or muscular pain, for example arthritis.

In another embodiment the particulate material is a ceramic of a size between 800 to 1000 micron and impregnated with an active ingredient, the particles are arranged in formation in one or more planes and bonded together and then adhered to the sheet material. It will be appreciated that by varying the manner in which the particles are bonded together the manner in which the active ingredient(s) is absorbed can be varied.

In one embodiment a size range of between 200-600 microns may be employed.

In another embodiment the particulate material is provided by spheres of copper, of 0.5mm diameter and arranged to surround in close formation a small magnet, or an array of magnetised particulate material, which arrangement may be bonded directly to the carrier or may be embedded in a flexible layer bonded to the carrier. The use of a magnet or magnetised particulate material assists in promoting the generation of electromagnetic impulse and/or current and/or magnetic field. Alternatively the particulate material material may comprise at least two different metals.

Any suitable magnet, whole or in particulate form may be used but preferably rare earth magnets, such as samarium or neodymium ion boron, are used.

Such magnets are powerful for their size and thus much small magnets can used to the same effect. In one embodiment a neodymium ion boron magnet of a size 3mm across by 1mm deep and a strength of 2000 oersteds is preferred.

The magnet or magnetic particles are preferably coated with a suitable material, e.g. zinc or plastics.

In another embodiment of the present invention a plurality of layers of spherical granules of copper, of a size between 45 up to 1000 micron and covering an area of 30mm square. When the resulting arrangement is applied to the skin the sweat from the skin is drawn up by the capillary action created between the granules which results in an improved circulation of the sweat. Hair on the skin can also be accommodated between the granules more readily. The arrangement may be gently massaged to provide or enhance pressure point stimulation by the granules on the skin. This arrangement may be used with a magnet as in other embodiments. The movement of copper particles in the magnetic field generates very small electric impulses.

In another embodiment porous copper spheres may be provided and can act a carrier for another active ingredient, or a catalyst for example.

Arrangements of these later embodiments can also provide a cushion or cushions to protect the wearer against injury from other components of the patch, such as a large magnet of the type described in GB Patent *1596314.*

The Invention will now be described by way of example with reference to the accompanying drawings in which:-
Figure 1 is an enlarged plan of a patch in accordance with one embodiment of the invention;
Figure 2 is a section of the line II-II of Figure 1; and
Figure 3 corresponds to Figure 1 but shows a patch in accordance with a further embodiment of the invention.

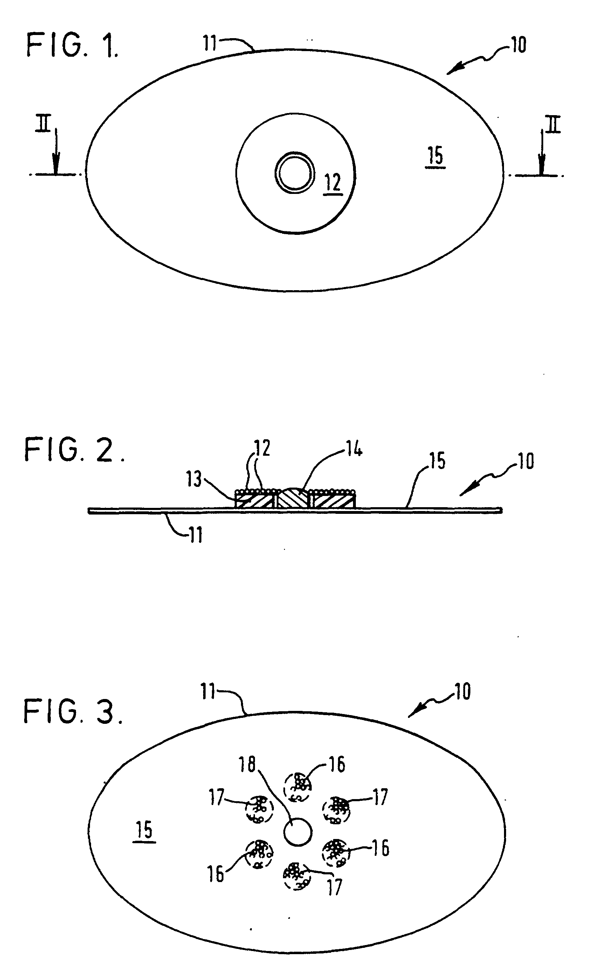

In Figures 1 and 2 a patch structure 10 for transdermal therapy comprises a layer of sheet material 11 carrying particulate material 12 of copper, held by an adhesive pad 13 in a ring formation about a zinc coated rare earth magnet 14. The sheet material 11 is provided with a further layer of adhesive 15 for application to the skin(not shown).

In the embodiment of Figure 3 the patch structure 10 is provided with a plurality of areas of zinc particles 16 and copper particles 17 arranged in formation so that the particle areas are in close proximity but do not touch, and surround a rare earth magnet 18. Again the sheet material carries an adhesive layer 15.

## Claims

1. A patch for transdermal therapy comprising at least one layer (11) of sheet material having on at least one surface thereof at least one magnet (14), the patch being **characterised by** particulate copper (12) or an alloy thereof on said one surface of said layer (11) in conjunction with said magnet (14).

2. A patch as claimed in Claim 1 **characterised by** particulate zinc or an alloy thereof in conjunction with said particulate copper (12) and said magnet (14).

3. A patch as claimed in Claim 1 **characterised in that** zinc is provided as a coating on said magnet (14).

4. A patch as claimed in Claim 2 wherein said particulate zinc and said particulate copper are mixed.

5. A patch as claimed in any one of the preceding claims **characterised in that** said magnet (14) is a rare earth magnet.

6. A patch as claimed in Claim 1 **characterised in that** said magnet (14) is coated with copper or an alloy thereof.

7. A patch as claimed in any one of the preceding claims **characterised in that** said magnet (14) is coated with plastics material.

8. A patch as claimed in any one of the preceding claims **characterised in that** said particulate material comprises particles having a rounded surface.

9. A patch as claimed in any one of the preceding claims **characterised in that** said layer (11) of sheet material defines a carrier for said particulate material and carries an adhesive (15) for securing the patch to the skin of the user.

## Patentansprüche

1. Pflaster für transdermale Therapie, das mindestens eine Lage (11) eines Schichtmaterials aufweist, das auf mindestens einer Oberfläche davon mindestens einen Magneten (14) aufweist, wobei das Pflaster durch teilchenförmiges Kupfer (12) oder eine Legierung davon auf der einen Oberfläche der Lage (11) in Verbindung mit dem Magneten (14) gekennzeichnet wird.

2. Pflaster nach Anspruch 1, **gekennzeichnet durch** teilchenförmiges Zink oder eine Legierung davon in Verbindung mit dem teilchenförmigen Kupfer (12) und dem Magneten (14).

3. Pflaster nach Anspruch 1, **dadurch gekennzeichnet, daß** das Zink als eine Beschichtung auf dem Magneten (14) vorhanden ist.

4. Pflaster nach Anspruch 2, bei dem das teilchenförmige Zink und das teilchenförmige Kupfer vermischt sind.

5. Pflaster nach einem der vorhergehenden Ansprüche, **dadurch gekennzeichnet, daß** der Magnet (14) ein Seltenerdmagnet ist.

6. Pflaster nach Anspruch 1, **dadurch gekennzeichnet, daß** der Magnet (14) mit Kupfer oder einer Legierung davon beschichtet ist.

7. Pflaster nach einem der vorhergehenden Ansprüche, **dadurch gekennzeichnet, daß** der Magnet (14) mit Kunststoffmaterial beschichtet ist

8. Pflaster nach einem der vorhergehenden Ansprüche, **dadurch gekennzeichnet, daß** das teilchenformige Material Teilchen mit einer abgerundeten Oberfläche aufweist

9. Pflaster nach einem der vorhergehenden Ansprüche, **dadurch gekennzeichnet, daß** die Lage (11) des Schichtmaterials einen Träger für das teilchenförmige Material definiert und einen Klebstoff (15) für das Sichern des Pflasters auf der Haut des Benutzers aufnimmt.

## Revendications

1. Timbre pour une thérapie transdermique comprenant au moins une couche (11) de matériau de feuille comportant sur au moins une surface correspondante au moins un aimant (14), le timbre étant **caractérisé par** un cuivre particulaire (12) ou un alliage correspondant sur ladite une surface de ladite couche (11) combiné audit aimant (14).

2. Timbre selon la revendication 1, **caractérisé par** du zinc particulaire ou un alliage correspondant combiné audit cuivre particulaire (12) et audit aimant (14).

3. Timbre selon la revendication 1, **caractérisé en ce que** le zinc est appliqué par revêtement sur ledit aimant (14).

4. Timbre selon la revendication 2, dans lequel ledit zinc particulaire et ledit cuivre particulaire sont mélangés.

5. Timbre selon l'une quelconque des revendications précédentes, **caractérisé en ce que** ledit aimant (14) est un aimant des terres rares.

6. Timbre selon la revendication 1, **caractérisé en ce que** ledit aimant (14) est revêtu de cuivre ou d'un alliage correspondant.

7. Timbre selon l'une quelconque des revendications précédentes, **caractérisé en ce que** ledit aimant (14) est revêtu d'un matériau pastique.

8. Timbre selon l'une quelconque des revendications précédentes, **caractérisé en ce que** ledit matériau particulaire comprend des particules ayant une surface arrondie.

9. Timbre selon l'une quelconque des revendications précédentes, **caractérisé en ce que** ladite couche (11) de matériau de feuille définit un support dudit matériau particulaire et supporte un adhésif (15) pour fixer le timbre sur la peau de l'utilisateur.
